# EUROPÄISCHE PATENTSCHRIFT

(11) **EP 4 008 232 B1**
(45) Veröffentlichungstag und Bekanntmachung des Hinweises auf die Patenterteilung: **23.04.2025**
(21) Anmeldenummer: 21206443.0
(22) Anmeldetag: 04.11.2021
(51) Int. Cl.: A47L 25/00, A61L 2/14

(54) **REINIGUNGSVERFAHREN VON BEKLEIDUNGSSTÜCKEN**
METHOD FOR CLEANING ARTICLES OF CLOTHING
PROCÉDÉ DE NETTOYAGE DE VÊTEMENTS

(30) Priorität: 01.12.2020 DE 102020215099
(43) Veröffentlichungstag der Anmeldung: 08.06.2022
(73) Patentinhaber: BSH Hausgeräte GmbH, 81739 München (DE)
(72) Erfinder: Schreiner, Dominik, 36043 Fulda (DE); Wald, Andreas, 36160 Dipperz (DE); Lange, Matthias, 98693 Ilmenau (DE)

(56) Entgegenhaltungen:
- CN-A- 108 771 767
- DE-A1- 102018 209 735
- DE-A1- 102018 213 144
- KR-A- 20120 079 523

## Beschreibung

Die Erfindung betrifft ein Verfahren zum Reinigen und/oder Erfrischen von Bekleidungsstücken mit Hilfe eines Plasmageräts durch einen Benutzer. Zudem betrifft die Erfindung ein Plasmagerät, insbesondere zum Reinigen und/oder Erfrischen von Bekleidungsstücken, aufweisend zumindest eine Plasmaquelle, zumindest einen Beschleunigungssensor und eine Kommunikationseinheit.

Geräte zum Reinigen von Bekleidungsstücken sind herkömmlicherweise bekannt, z.B. aus der DE 10 2018 213 144 A1. Hierzu wird ein zu reinigendes Bekleidungsstück im Liegen oder im tragenden Zustand mit dem Gerät oberflächlich abgefahren. Jedoch ist es dem Benutzer nicht möglich zu sehen, wo das Bekleidungsstück bereits gereinigt beziehungsweise erfrischt worden ist. Diese Information muss sich der Benutzer bislang merken. Dies kann die Folge haben, dass bestimmte Bereiche des Bekleidungsstücks zu oft oder gar nicht erfrischt worden sind.

Aufgabe der Erfindung ist es, beim Reinigen oder Erfrischen von Bekleidungsstücken mit einem Plasmagerät die oben genannten Nachteile nach dem Stand der Technik zu vermeiden und insbesondere eine flächendeckende Reinigung beziehungsweise Erfrischung zu gewährleisten.

Diese Aufgabe wird durch ein Verfahren zum Reinigen und/oder Erfrischen von Bekleidungsstücken mit Hilfe eines Plasmageräts durch einen Benutzer mit den Merkmalen des Anspruchs 1 und durch ein Plasmagerät mit den Merkmalen des Anspruchs 10 gelöst. Vorteilhafte Ausgestaltungen und Weiterbildungen sind Gegenstand der Unteransprüche.

Erfindungsgemäß umfasst ein Verfahren zum Reinigen und/oder Erfrischen von Bekleidungsstücken mit Hilfe eines Plasmageräts durch einen Benutzer folgende Schritte:
- Auswahl eines zu reinigenden Bekleidungsstücks oder Scannen des zu reinigenden Bekleidungsstücks mittels einer externen Anzeigeeinheit durch den Benutzer,
- Festlegen eines Startpunkts eines gewünschten Reinigungsbereichs und/oder Reinigungswegs,
- Beginn der Reinigung und/oder des Erfrischens des Bekleidungsstücks,
- Anzeigen von gereinigten und/oder erfrischten Bereiche des Bekleidungsstücks an der externen Anzeigeeinheit.

Die erfindungsgemäße Lösung zeichnet sich dadurch aus, dass bereits erfrischte beziehungsweise gereinigte Bereiche des zu reinigenden Bekleidungsstücks dem Benutzer an der externen Anzeigeeinheit visuell dargestellt werden. Insbesondere kann der Benutzer seine bereits erfrischten Bereiche an der Anzeigeeinheit überprüfen und eventuell seine Reinigung beziehungsweise Erfrischung optimieren. Ein mehrfaches Reinigen bestimmter Bereiche oder gar ein Vergessen beziehungsweise Auslassen von Bereichen des Bekleidungsstücks können so vorteilhafterweise vermieden werden.

Vor Beginn der Reinigung wird insbesondere das zu reinigende Bekleidungsstück identifiziert, um eine optimale und vollständige Reinigung beziehungsweise Erfrischung gewährleisten zu können. Hierzu kann beispielsweise das zu bearbeitende Bekleidungsstück mit der externen Anzeigeeinheit, insbesondere über eine entsprechende App auf dem Handy des Benutzers, eingescannt werden. Alternativ ist es möglich, dass die App des Handys des Benutzers eine Vorwahl an unterschiedlichen Bekleidungsstücken wie beispielsweise T-Shirt, Hose, Sweatshirt oder ähnliches vorgibt, aus der der Benutzer nun das entsprechende zu bearbeitende Bekleidungsstück selektieren kann. Anschließend wird vorzugsweise das zu bearbeitende Bekleidungsstück an der externen Anzeigeeinheit, insbesondere an der App des Handys, angezeigt.

Nun wird der Startpunkt des Reinigungsbereichs oder des Reinigungswegs durch den Benutzer oder wiederum durch beispielsweise die App der externen Anzeigeeinheit, also des Handys, festgelegt. Von diesem Startpunkt ausgehend wird nun der Reinigungsbereich oder der Reinigungsweg mit dem Plasmagerät abgefahren.

Die Bearbeitung des Bekleidungsstücks beginnt nun nach erfolgreicher Betätigung eines entsprechenden Startsignals an der externen Anzeigeeinheit. Beispielsweise wird in der App des Handys durch den Benutzer ein Start-Button gedrückt, sodass die Reinigung beginnt. Hierzu wird das Plasmagerät auf das Bekleidungsstück am Startpunkt aufgesetzt, wodurch das Plasma zündet.

Durch das erfindungsgemäße Reinigungsverfahren kann ein effizientes Nutzen des Plasmageräts gewährleistet werden. Eine vollständige Abdeckung der zu reinigenden Bereiche des Bekleidungsstücks ist mit Vorteil möglich. Zusätzlich ist es möglich, beispielsweise eine Statistikauswertung über die externe Anzeigeeinheit und insbesondere über die App bereitzustellen. Beispiele für solche Statistikauswertungen sind unter anderem:
- Du benötigst vier Überfahrten bis zu einem guten Ergebnis,
- du hast bereits zwanzig Hemden und acht T-Shirts erfrischt,
- du hast vier Kleider und sieben T-Shirts in 33 Minuten erfrischt,
- du hast vier Kleider und sieben T-Shirts mit circa 8 m² Fläche in 33 Minuten erfrischt.

Durch das erfindungsgemäße Reinigungsverfahren erhält der Benutzer vorteilhafterweise ein optimales Feedback über die Reinigung und insbesondere die Qualität der Reinigung. Beispielsweise erhält der Benutzer Hinweise über vergessene Bereiche, Hinweise beziehungsweise visuelle und/oder akustische Warnungen bei zu häufigem Überstreichen eines Bereichs und/oder eine Warnung bei Stillstand und eingeschalteter Plasmaquelle. Auch ist es möglich, dem Benutzer über die externe Anzeigeeinheit Behandlungsvorschläge zukommen beziehungsweise anzeigen zu lassen.

Auch ist ein Vernetzen beispielsweise der App mit einer sogenannten HomeConnect Applikation möglich. Die HomeConnect Applikation ist insbesondere eine Applikation, die Daten, Anwendungen etc. einer Mehrzahl von Haushaltsgeräten des oder der Benutzer eines Haushalts sammelt, anzeigt beziehungsweise steuert. Dies ist vorzugsweise über eine Bluetooth Verbindung oder ein W-Lan realisierbar, beispielsweise über das COM-Modul beziehungsweise System Master, um eine schnelle Datenübertragung zu gewährleisten.

Unter einem Plasmagerät ist insbesondere jegliches Gerät zu verstehen, dass eine Plasmaquelle zum Erzeugen eines Plasmas aufweist. Insbesondere dient das Plasmagerät, das in Fachkreisen auch unter dem Begriff Plasma Refresher bekannt ist, mit seinem Plasma zur flächigen Behandlung von Oberflächen, insbesondere von Textilien oder ähnlichen Materialien. Mit dem Plasmagerät können demzufolge derartige Oberflächenbereiche geruchsneutralisiert werden. Dafür wird bevorzugt kaltes Atmosphärendruckplasma verwendet.

Plasma wird neben den drei Aggregatzuständen fest, flüssig oder gasförmig als vierter Aggregatzustand bezeichnet. Wird einem Gas oder Gasgemisch hinreichend viel Energie, beispielsweise in Form von elektrischer Energie, zugeführt, so werden einige Atome des Gases ionisiert, d.h. Elektronen werden aus der Atomhülle entfernt und bewegen sich als freie Teilchen, sodass ein positiv geladenes Atom zurückbleibt. Wenn ein Gas aus einem ausreichend hohen Anteil an freien Ionen und Elektronen besteht, so bezeichnet man den Aggregatzustand als Plasma. Plasma ist also Materie, dessen Bestandteile teilweise geladene Komponenten, Ionen und Elektronen sind, die sich als freie Ladungsträger bewegen.

Nichtthermische Plasma, das auch als kaltes Plasma bezeichnet wird, kann gezielt zur Beseitigung von Gerüchen und bestimmten Kohlenwasserstoffen eingesetzt werden, indem es in einem geschlossenen Gehäuse zur Ozon-Erzeugung genutzt wird, das dann wiederum wirksam ist, beispielsweise im Bereich der Automobil-Aufbereitung oder bei der Ozon-Wäsche (Active Oxygen). Weiterhin finden nichtthermische Plasmen Anwendung in der Medizintechnik, zum Beispiel bei der Behandlung von schlecht oder nicht heilenden Wunden mit Hilfe eines sogenannten Plasmastifts, indem auf die antimikrobielle Wirkung von "kaltem Plasma" zurückgegriffen wird.

Dass Plasmen geruchsinaktivierende und antimikrobielle Eigenschaften besitzen, ist bekannt. Die Ursachen der antibakteriellen Wirkung eines Plasmas liegen in Hitze, Austrocknung, Scherspannung, UV-Strahlung, freie Radikale und Ladungen. Bei Niederdruckplasmen, die auch kalte Plasmen genannt werden, spielt die Hitze eine untergeordnete Rolle, da diese Plasmen bei Raumtemperatur betrieben werden. In solchen Niederdruckplasmen entstehen besonders reaktive Partikel, wie beispielsweise verschiedene Sauerstoff- oder Stickstoffspezies, die eine ausreichend hohe Lebensdauer aufweisen, um bei einer indirekten Exposition organische Verbindungen zu schädigen. Zu diesen Partikeln zählen unter anderem atomarer Sauerstoff, Superoxidradikale, Ozon, Hydroxylradikale, Stickstoffmonooxid und Stickstoffdioxid. Diese Partikel zeigen eine zerstörerische Wirkung auf unterschiedlichste Geruchskomponenten wie auch Zellkomponenten.

Werden Geruchskomponenten, welche meist aus Kohlenstoffverbindungen bestehen, wie auch Zellwände von Bakterien, Keimen, Viren, Pilzen oder anderen vergleichbaren Mikroorganismen dem Plasma direkt ausgesetzt, so laden sich diese aufgrund des Beschusses mit den im Plasma vorhandenen Elektronen negativ auf. Aufgrund der elektrostatischen Abstoßung führt dies zu mechanischen Spannungen bis hin zur Überschreitung der Zugfestigkeit und der Zerstörung von dem Geruchsmolekül, bzw. der Zellwand. Aber nicht nur mechanische Verspannungen aufgrund der Ladung können die Zellwände zerstören, sondern auch die Störung des Ladungsgleichgewichts der Geruchsmoleküle, bzw. der Zellwand durch verschiedene, weitere elektrostatische Wechselwirkungen und der Elektrolyse, z. B. durch Änderung der Permeabilität der Zellwände. Ein Mechanismus zur Inaktivierung von Mikroorganismen ergibt sich auch aus den sehr energiereichen Ionen. Mittels einer Hochfrequenz kann das Plasma erzeugt werden.

Niederdruckplasmen sind daher besonders gut zur Inaktivierung von Gerüchen an textilem Gewebe oder von haushaltsüblichen Oberflächen oder dergleichen geeignet, um eine Geruchsaktivierung zu erreichen.

Bei der Verwendung einer Plasmaquelle ist vorzugsweise auf eine damit verbundene Ozonbildung zu achten. Ozon ist ein aus drei Sauerstoffatomen (O) aufgebautes Molekül (O₃). Aus dem kurzlebigen Ozonmolekül spaltet sich ein Sauerstoffatom (O) ab, das bevorzugt mit den Geruchsmolekülen oder Kohlenstoffverbindung reagiert und diese chemisch verändert. Damit bei einer aktivierten Plasmaquelle keine übermäßige Ozonproduktion stattfindet, ist auf einen direkten Kontakt mit der textilen Oberfläche zu achten. Weiter soll die Plasmaquelle von dem Luftsauerstoff soweit abgeschirmt werden, dass zwar genügend Sauerstoff zur Ozonproduktion zur Verfügung steht, andererseits ist eine oberseitige Abschirmung von Luftsauerstoff an der Textiloberfläche wünschenswert, da noch genügend Luftsauerstoff über die Unterseite durch das Textil dringen kann. Daher werden Mittel vorgeschlagen die einen Kontakt mit dem Luftsauerstoff an der Oberseite begrenzen. Dies können Schutzwände aus einem interten Gas sein. Weiter kann eine Reduzierung der Faltenbildung ebenfalls eine erhöhte Ozonproduktion an der Plasmaquelle vermeiden.

Der Abstand der Plasmaquelle zu der behandelnden Oberfläche beträgt vorzugsweise 0 mm. Damit geht eine verringerte Ozonkonzentration einher. Ein Abstand zwischen 0 mm und 1 mm trägt zu einer höheren Ozonkonzentration bei, falls dies gewünscht wird.

Unter einem Bekleidungsstück im Sinne der Anmeldung ist jegliche von Geruchsstoffen zu befreiende Oberfläche zu verstehen, die ausgewählt ist aus den textilen Materialien wie natürliche, pflanzlich wie tierische (Naturfasern wie z. B. Baum-, Schafwolle, Seide, Leinen, Filz) oder künstliche Kleiderstoffe (Chemiefasern wie z. B. Nylon). Weiter umfasst der Begriff Materialien und Gegenstände aus Keramik, Kunststoff, Federn, Leder, Glas, Holz, oder Metall.

Unter Reinigen beziehungsweise Erfrischen ist vorliegend eine Geruchsneutralisierung zu verstehen. Insbesondere Gerüche, die als unangenehm empfundenen werden können, wie beispielsweise Buttersäure, organische, der Buttersäure ähnliche Säuren, Schweiß, festgesetzter Zigarettenrauch u. ä., sollen mit dem Reinigungsverfahren aktiv beseitigt werden und/oder mittels nicht unangenehmer Gerüche, wie zum Beispiel Parfüm, überdeckt werden. Weiter können bei dem Reinigungsverfahren sogenannte nichtgeruchsrelevante Moleküle beseitigt oder zerstört werden, vorzugsweise Allergene, Proteinmoleküle, Prionen u. ä.

Unter einem Startpunkt ist jeglicher Punkt zu verstehen, an dem das Reinigen oder Erfrischen des zu reinigenden Bekleidungsstücks begonnen werden kann. Hierbei ist selbstverständlich, dass der Startpunkt kein Punkt im mathematischen Sinn sein muss. Beispielsweise kann der Startpunkt auch ein Startbereich sein.

Unter einer externen Anzeigeeinheit ist vorliegend insbesondere jegliche Einheit zu verstehen, die außerhalb des Plasmageräts angeordnet und zu einer Anzeige, Bereitstellung, Übermittlung und/oder Übertragung von Daten geeignet ist, wie beispielsweise ein Handy, ein Tablet, ein Touchpad und/oder ein Computer beziehungsweise Laptop.

Gemäß einer bevorzugten Ausgestaltung wird eine Kommunikation und/oder ein Datenaustausch zwischen dem Plasmagerät und der externen Anzeigeeinheit durchgeführt. Hierzu fungiert das Plasmagerät insbesondere als Kommunikationseinheit. Unter einer Kommunikationseinheit ist vorliegend insbesondere jegliche Einheit zu verstehen, die unter anderem als Kommunikationseinheit zur Übermittlung und Übertragung von Daten und/oder Parameter dienen kann. Das Plasmageräts ist also geeignet, mit der externen Anzeigeeinheit zu kommunizieren. Das Plasmagerät kann hierzu einen Speicher für die zu übermittelten Daten aufweisen.

Gemäß einer weiteren bevorzugten Ausgestaltung wird der Startpunkt automatisch von der externen Anzeigeeinheit festgelegt. Zum Beispiel zeigt die externe Anzeigeeinheit nach der Auswahl des zu bearbeitenden Bekleidungsstücks den gewünschten Startpunkt an, beispielsweise rechts unten am Hemd. Alternativ wird der Startpunkt manuell von dem Benutzer an der externen Anzeigeeinheit festgelegt. Hierzu tippt der Benutzer beispielsweise nach Festlegen des zu bearbeitenden Bekleidungsstücks auf eine Skizze oder ein Bild an der externen Anzeigeeinheit und insbesondere an der App und legt den Startpunkt entsprechend fest. Dadurch kann insbesondere der automatisch generierte Startpunkt überschrieben werden.

Gemäß einer weiteren bevorzugten Ausgestaltung beginnt der Benutzer durch eine Eingabe an der externen Anzeigeeinheit und/oder durch ein Aufsetzen des Plasmageräts am Startpunkt die Reinigung und/oder das Erfrischen.

Gemäß einer weiteren bevorzugten Ausgestaltung werden mit dem Plasmagerät dessen Bewegungsrichtung und/oder Bewegungsgeschwindigkeit detektiert und/oder die beim Reinigen und/oder Erfrischen zurückgelegte Entfernung und/oder Richtung ermittelt. Hierzu weist das Plasmagerät vorzugsweise einen Beschleunigungssensor auf. Dieser kann nun Bewegungen des Plasmageräts detektieren und ebenso die zurückgelegte Entfernung und Richtung ermitteln. Eine detailgetreue und optimale Darstellung der bereits getätigten Reinigung beziehungsweise Erfrischung kann so mit Vorteil gewährleistet werden.

Gemäß einer weiteren bevorzugten Ausgestaltung werden zudem mit dem Plasmagerät und insbesondere mit dem integrierten Beschleunigungssensor eine Richtungsumkehr und/oder ein Abheben des Plasmageräts vom Bekleidungsstück detektiert, wodurch die Reinigung und insbesondere dessen Darstellung weiter optimiert werden kann.

Gemäß einer weiteren bevorzugten Ausgestaltung werden die gereinigten und/oder erfrischten Bereiche des Bekleidungsstücks an der externen Anzeigeeinheit farblich dargestellt. Beispielsweise werden die bearbeiteten Bereiche an der ausgewählten oder eingescannten Skizze des Bekleidungsstücks farblich hinterlegt, beispielsweise blau dargestellt. Noch nicht gereinigte Bereiche können so dem Benutzer angezeigt werden.

Gemäß einer weiteren bevorzugten Ausgestaltung können an der externen Anzeigeeinheit Bereiche an dem Bekleidungsstück festgelegt werden, die eine zusätzliche Reinigung und/oder Erfrischung erfahren sollen. Insbesondere kann der Benutzer dadurch sogenannte "Points of Interest" definieren, die beispielsweise eine besonders intensive Bearbeitung benötigen, wie zum Beispiel bei T-Shirts Regionen unter den Achseln. Hierzu kann bevorzugt mittels des Beschleunigungssensors des Plasmageräts ein Reinigungsgitter erzeigt werden, das zum Beispiel eine gekrümmte Fläche darstellt und an der externen Anzeigeeinheit, insbesondere der App, auf den "Point of Interest" projiziert wird, um anzuzeigen, dass hier das Bekleidungsstück zusätzlich eine Reinigung beziehungsweise Erfrischung erfahren hat.

Gemäß einer weiteren bevorzugten Ausgestaltung wird mit dem Plasmagerät eine Positionsbestimmung am Bekleidungsstück durchgeführt. Hierzu findet beispielsweise der Beschleunigungssensor des Plasmageräts Anwendung. Alternativ kann die Positionsbestimmung auch über eine in dem Plasmagerät integrierte Kamera erfolgen, bei der ein Rand des Bekleidungsstücks als Grenze gewertet wird. Dies erfordert eine große Datenbank an Referenzbildern, sowie einen kontrastreichen Hintergrund zum Bekleidungsstück.

Gemäß einer weiteren bevorzugten Ausgestaltung kommuniziert das Plasmagerät mit dem Benutzer. Beispielsweise kann das Plasmagerät bei einer vollständig durchgeführten Bearbeitung des Bekleidungsstücks vorzugsweise grün aufleuchten, um den Benutzer zu signalisieren, dass das Bekleidungsstück ausreichend bearbeitet ist. Zusätzlich kann das Plasmagerät beispielsweise bei einer fehlerhaften Bearbeitung und/oder einer Störung vorzugsweise rot aufleuchten.

Erfindungsgemäß weist ein Plasmagerät, insbesondere zum Reinigen und/oder Erfrischen von Bekleidungsstücken, zumindest eine Plasmaquelle, zumindest einen Beschleunigungssensor und eine Kommunikationseinheit auf, wobei der Beschleunigungssensor eine Bewegungsrichtung und/oder Bewegungsgeschwindigkeit des Plasmageräts detektiert, die über die Kommunikationseinheit an eine externe Instanz sendbar sind, um gereinigte Bereiche des Bekleidungsstücks an der externen Instanz anzeigen zu lassen.

Jegliche Merkmale, Ausgestaltungen, Ausführungsformen und Vorteile das Verfahren betreffend finden auch in Zusammenhang mit dem erfindungsgemäßen Plasmagerät Anwendung, und umgekehrt.

Die Erfindung wird anhand der nachfolgenden, lediglich Beispiele darstellenden Ausführungen der Erfindung näher erläutert. Es zeigen:
- Figur 1:: eine schematische Ansicht eines Ausführungsbeispiels eines erfindungsgemäßen Verfahrens zum Reinigen und/oder Erfrischen von Bekleidungsstücken mit Hilfe eines Plasmageräts,
- Figur 2:: schematische Ansichten eines Ausführungsbeispiels eines erfindungsgemäßen Plasmageräts in verschiedenen kommunizierenden Modi, und
- Figur 3:: eine weitere schematische Ansicht eines Ausführungsbeispiels eines erfindungsgemäßen Verfahrens zum Reinigen und/oder Erfrischen von Bekleidungsstücken mit Hilfe eines Plasmageräts.

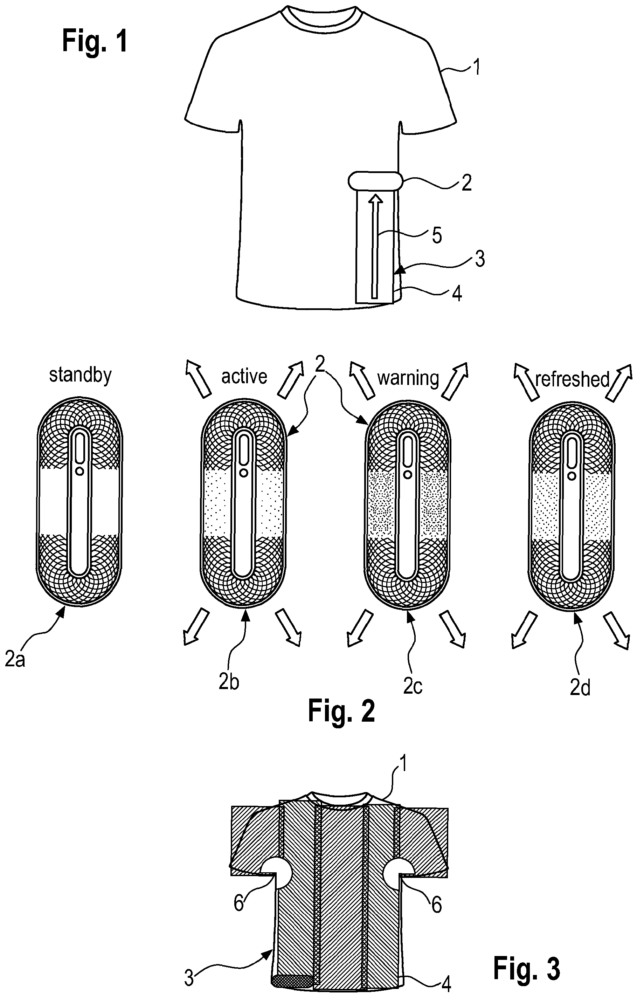

Figur 1 zeigt ein zur Bearbeitung, insbesondere Reinigung vorgesehenes Bekleidungsstück 1. Vorliegend ist das Bekleidungsstück 1 ein zu reinigendes T-Shirt. Zum Reinigen dieses T-Shirts findet vorliegend ein Plasmagerät 2, insbesondere ein sogenannter Plasmarefresher Anwendung. Damit der Benutzer nun seine bereits gereinigten Bereiche 3 am T-Shirt visuell nachverfolgen kann, werden erfindungsgemäß diese gereinigten Bereiche 3 über eine App dargestellt. So ist der Benutzer in der Lage, seinen bereits gereinigten Bereich 3 in dieser App zu überprüfen.

Hierzu öffnet der Benutzer vor der Bearbeitung des Bekleidungsstücks 1 seine sogenannte HomeConnect App und definiert sein Bekleidungsstück 1. Dies kann dadurch stattfinden, dass der Benutzer mit dem Plasmagerät 2 das Bekleidungsstück 1 einscannt. Alternativ kann der Benutzer das Bekleidungsstück 1 beispielsweise als T-Shirt in einer Vorauswahl in der App selektieren. Die Form beziehungsweise Skizze des eingescannten oder selektierten Bekleidungsstücks 1 wird anschließend in der App angezeigt.

Ein Startpunkt 4 zum Reinigen kann durch die App festgelegt werden, beispielsweise rechts unten am T-Shirt. Alternativ kann der Benutzer manuell den gewünschten Startpunkt 4 festlegen, indem er in der App auf die Skizze und den gewünschten Startpunkt 4 tippt.

Zum Starten der Reinigung wird anschließend in der App der Startknopf beziehungsweise Button betätigt. Durch das Aufsetzen des Plasmageräts 2 an dem Startpunkt 4 des Bekleidungsstücks 1 wird das Plasma gezündet. Manuell wird das Plasmagerät 2 entlang des gewünschten Reinigungswegs 5 oder Reinigungsbereichs an dem T-Shirt entlanggeführt. Mittels eines Beschleunigungssensors im Plasmagerät 2 wird die Bewegung des Plasmageräts 2 detektiert. Darüber hinaus wird die beim Reinigen zurückgelegte Entfernung und Richtung ermittelt. Auch eine Richtungsumkehr oder ein Abheben des Plasmageräts 2 kann mit dem Beschleunigungssensor detektiert werden. Parallel wird der bereits gereinigte Bereich 3 in der App farblich dargestellt. Beispielsweise färbt sich der bereits gereinigte Bereich 3 des T-Shirts in der App blau.

Alternativ zu dem Beschleunigungssensor kann eine Positionsbestimmung des Plasmageräts 2 am T-Shirt auch über eine Kamera erfolgen, bei der immer ein Rand des Bekleidungsstücks 1 als Grenze gewertet wird. Dies erfordert neben der im Plasmagerät 2 integrierten Kamera eine Datenbank an Referenzbildern, sowie einen kontrastreichen Hintergrund zum Bekleidungsstück 1.

Zur Umsetzung dieser App-Anzeige des erfrischten Bereichs 3 ist eine Kommunikation zwischen Plasmagerät 2 und App (beispielsweise HomeConnect App) notwendig. Diese kann beispielsweise über eine Bluetooth Verbindung, ein W-Lan, ein COM-Modul beziehungsweise System Master für eine schnelle Datenübertragung realisiert werden.

Durch die visuelle Darstellung der gereinigten Bereiche 3 ist ein optimaler Nutzen des Plasmageräts 2 einfach möglich. Zusätzlich ist eine statistische Auswertung über die App möglich. Beispielsweise kann die App anzeigen, wie viele Bekleidungsstücke insgesamt gereinigt wurden, wie viele Behandlungen bis zu einem optimalen Ergebnis notwendig waren und/oder wie lange beziehungsweise wie viel Fläche insgesamt gereinigt wurde. Ein verbessertes Feedback ermöglicht sich über die App. Beispielsweise können in der App Hinweise angezeigt werden, die beispielsweise vergessene Bereiche, visuelle und/oder akustische Warnungen bei zu häufigem Überstreichen eines Bereichs, oder Warnungen bei Stillstand und eingeschalteter Plasmaquelle betreffen. Auch Behandlungsvorschläge können dem Benutzer in der App angeboten werden.

In Figur 2 ist ein mögliches Plasmagerät 2 zum Reinigen und/oder Erfrischen von Bekleidungsstücken 1 in verschiedenen Behandlungsmodi 2a bis 2d dargestellt. Derartige Plasmageräte 2 weisen unter anderem eine Plasmaquelle, einen Beschleunigungssensor und eine Kommunikationseinheit auf. Der Beschleunigungssensor detektiert im Betrieb eine Bewegungsrichtung und/oder Bewegungsgeschwindigkeit des Plasmageräts 2, die über die Kommunikationseinheit an eine externe Instanz wie beispielsweise ein Handy des Benutzers gesendet werden, um gereinigte Bereiche des Bekleidungsstücks an der App des Handys anzeigen zu lassen.

Neben der App ist es möglich, dass das Plasmagerät 2 an sich mit dem Benutzer kommuniziert. Beispielsweise kann das Plasmagerät in einem standby-Modus 2a weiß leuchten oder unbeleuchtet sein. In einem aktiven Modus 2b leuchtet das Plasmagerät blau. Tritt eine Warnung auf, kann das Plasmagerät rot leuchten (Warn-Modus 2c). Ist das Bekleidungsstück vollständig gereinigt beziehungsweise behandelt, kann das Plasmagerät 2 grün leuchten (refreshed-Modus 2d).

In Figur 3 ist die in der App dargestellte Skizze des T-Shirts aus dem Ausführungsbeispiel der Figur 1 mit vollständig gereinigten Bereichen 3 gezeigt. Hier sind zudem sogenannte Points of Interest 6 vom Benutzer in der App definiert. Diese Points of Interest 6 erfahren bei der Reinigung des T-Shirts eine zusätzliche Behandlung, zum Beispiel Erfrischung, und sind vom Benutzer manuell festgelegt worden. Beispielsweise betreffen diese Points of Interest 6 Regionen unter den Achseln des T-Shirts. Hier kann mittels des Beschleunigungssensors ein zusätzliches Gitter (CleaningGrid) erzeugt werden, das zum Beispiel eine gekrümmte Fläche darstellt und in der App auf den Point of Interest 6 projiziert wird, um anzuzeigen, dass an dieser Stelle beziehungsweise an diesen Stellen das Bekleidungsstück 1 zusätzlich erfrischt wurde.

## Patentansprüche

1. Verfahren zum Reinigen und/oder Erfrischen von Bekleidungsstücken (1) mit Hilfe eines Plasmageräts (2) durch einen Benutzer, **gekennzeichnet durch** folgende Schritte:
- Auswahl eines zu reinigenden Bekleidungsstücks (1) oder Scannen des zu reinigenden Bekleidungsstücks (1) mittels einer externen Anzeigeeinheit durch den Benutzer,
- Festlegen eines Startpunkts (4) eines gewünschten Reinigungsbereichs (3) und/oder Reinigungswegs (5),
- Beginn der Reinigung und/oder des Erfrischens des Bekleidungsstücks (1),
- Anzeigen von gereinigten und/oder erfrischten Bereiche (3) des Bekleidungsstücks (1) an der externen Anzeigeeinheit.

2. Verfahren nach Anspruch 1, wobei eine Kommunikation und/oder ein Datenaustausch zwischen dem Plasmagerät (2) und der externen Anzeigeeinheit durchgeführt wird.

3. Verfahren nach einem der vorhergehenden Ansprüche, wobei der Startpunkt (4) automatisch von der externen Anzeigeeinheit oder manuell von dem Benutzer festgelegt wird.

4. Verfahren nach einem der vorhergehenden Ansprüche, wobei der Benutzer durch eine Eingabe an der externen Anzeigeeinheit und/oder durch ein Aufsetzen des Plasmageräts (2) am Startpunkt (4) die Reinigung und/oder das Erfrischen beginnt.

5. Verfahren nach einem der vorhergehenden Ansprüche, wobei mit dem Plasmagerät (2) dessen Bewegungsrichtung und/oder Bewegungsgeschwindigkeit detektiert und/oder die beim Reinigen und/oder Erfrischen zurückgelegte Entfernung und/oder Richtung ermittelt werden.

6. Verfahren nach einem der vorhergehenden Ansprüche, wobei mit dem Plasmagerät (2) eine Richtungsumkehr und/oder ein Abheben des Plasmageräts (2) vom Bekleidungsstück (1) detektiert werden.

7. Verfahren nach einem der vorhergehenden Ansprüche, wobei die gereinigten und/oder erfrischten Bereiche (3) des Bekleidungsstücks (1) an der externen Anzeigeeinheit farblich dargestellt werden.

8. Verfahren nach einem der vorhergehenden Ansprüche, wobei an der externen Anzeigeeinheit Bereiche (6) an dem Bekleidungsstück (1) festgelegt werden können, die eine zusätzliche Reinigung und/oder Erfrischung erfahren sollen.

9. Verfahren nach einem der vorhergehenden Ansprüche, wobei mit dem Plasmagerät (2) eine Positionsbestimmung am Bekleidungsstück (1) durchgeführt wird.

10. Plasmagerät (2), insbesondere zum Reinigen und/oder Erfrischen von Bekleidungsstücken (1), aufweisend zumindest eine Plasmaquelle, zumindest einen Beschleunigungssensor, wobei der Beschleunigungssensor eine Bewegungsrichtung und/oder Bewegungsgeschwindigkeit des Plasmageräts (2) detektiert, **dadurch gekennzeichnet, dass** das Plasmagerät (2) eine Kommunikationseinheit aufweist und dass die detektierte Bewegungsrichtung und/oder Bewegungsgeschwindigkeit des Plasmageräts (2) über die Kommunikationseinheit an eine externe Instanz sendbar sind/ist, um gereinigte Bereiche (3) des Bekleidungsstücks (1) an der externen Instanz anzeigen zu lassen.

## Claims

1. Method for cleaning and/or refreshing items of clothing (1) with the aid of a plasma appliance (2) by way of a user, **characterised by** the following steps:
- selecting an item of clothing (1) to be cleaned and/or scanning the item of clothing (1) to be cleaned by means of an external display unit by way of the user,
- specifying a start point (4) of a desired cleaning region (3) and/or cleaning path (5),
- beginning the cleaning and/or the refreshing of the item of clothing (1),
- displaying cleaned and/or refreshed regions (3) of the item of clothing (1) on the external display unit.

2. Method according to claim 1, wherein a communication and/or a data exchange is performed between the plasma appliance (2) and the external display unit.

3. Method according to one of the preceding claims, wherein the start point (4) is specified automatically by the external display unit or manually by the user.

4. Method according to one of the preceding claims, wherein the user begins the cleaning and/or the refreshing by way of an input on the external display unit and/or by positioning the plasma appliance (2) at the start point (4).

5. Method according to one of the preceding claims, wherein using the plasma appliance (2), the movement direction and/or movement speed thereof is/are detected and/or the distance covered and/or direction during the cleaning and/or refreshing is/are ascertained.

6. Method according to one of the preceding claims, wherein using the plasma appliance (2), a reversal of direction and/or a withdrawing of the plasma appliance (2) from the item of clothing (1) is/are detected.

7. Method according to one of the preceding claims, wherein the cleaned and/or refreshed regions (3) of the item of clothing (1) are represented in colour on the external display unit.

8. Method according to one of the preceding claims, wherein it is possible to specify, on the external display unit, regions (6) on the item of clothing (1) that are to experience additional cleaning and/or refreshing.

9. Method according to one of the preceding claims, wherein a position determination on the item of clothing (1) is performed using the plasma appliance (2).

10. Plasma appliance (2), in particular for cleaning and/or refreshing items of clothing (1), having at least one plasma source, at least one acceleration sensor, wherein the acceleration sensor detects a movement direction and/or movement speed of the plasma appliance (2), **characterised in that** the plasma appliance (2) has a communication unit and the detected movement direction and/or movement speed of the plasma appliance (2) can be sent to an external entity via the communication unit, in order to allow cleaned regions (3) of the item of clothing (1) to be displayed on the external entity.

## Revendications

1. Procédé de nettoyage et/ou de rafraîchissement d'articles vestimentaires (1) au moyen d'un appareil à plasma (2) par un utilisateur, **caractérisé par** les étapes suivantes :
- sélection d'un article vestimentaire à nettoyer (1) ou balayage de l'article vestimentaire à nettoyer (1) au moyen d'une unité d'affichage externe par l'utilisateur,
- spécification d'un point de départ (4) d'une zone de nettoyage souhaitée (3) et/ou d'un trajet de nettoyage (5),
- début du nettoyage et/ou du rafraîchissement de l'article vestimentaire (1),
- affichage des zones (3) nettoyées et/ou rafraîchies de l'article vestimentaire (1) sur l'unité d'affichage externe.

2. Procédé selon la revendication 1, dans lequel une communication et/ou un échange de données est effectué entre l'appareil à plasma (2) et l'unité d'affichage externe.

3. Procédé selon l'une des revendications précédentes, dans lequel le point de départ (4) est déterminé automatiquement par l'unité d'affichage externe ou manuellement par l'utilisateur.

4. Procédé selon l'une des revendications précédentes, dans lequel l'utilisateur démarre le nettoyage et/ou le rafraîchissement par une entrée sur l'unité d'affichage externe et/ou par une mise en place de l'appareil à plasma (2) au point de départ (4).

5. Procédé selon l'une des revendications précédentes, dans lequel, au moyen de l'appareil à plasma (2), sa direction de déplacement et/ou sa vitesse de déplacement sont détectées et/ou la distance parcourue et/ou la direction lors du nettoyage et/ou du rafraîchissement sont déterminées.

6. Procédé selon l'une des revendications précédentes, dans lequel, au moyen de l'appareil à plasma (2), un changement de direction et/ou un retrait de l'appareil à plasma (2) vis-à-vis de l'article vestimentaire (1) sont détectés.

7. Procédé selon l'une des revendications précédentes, dans lequel les zones nettoyées et/ou rafraîchies (3) de l'article vestimentaire (1) sont représentées en couleur sur l'unité d'affichage externe.

8. Procédé selon l'une des revendications précédentes, dans lequel des zones (6) sur l'article vestimentaire (1), qui doivent subir un nettoyage et/ou rafraîchissement supplémentaire, peuvent être spécifiées sur l'unité d'affichage externe.

9. Procédé selon l'une des revendications précédentes, dans lequel une détermination de position sur l'article vestimentaire (1) est exécutée au moyen de l'appareil à plasma (2).

10. Appareil à plasma (2), en particulier pour nettoyer et/ou rafraîchir des articles vestimentaires (1), comprenant au moins une source de plasma, au moins un capteur d'accélération,
dans lequel le capteur d'accélération détecte une direction de déplacement et/ou une vitesse de déplacement de l'appareil à plasma (2),
**caractérisé en ce que** l'appareil à plasma (2) comprend une unité de communication et **en ce que** la direction de déplacement et/ou la vitesse de déplacement détectée de l'appareil à plasma (2) peut (peuvent) être envoyées à une entité externe via l'unité de communication, afin d'afficher des zones nettoyées (3) de l'article vestimentaire (1) sur l'entité externe.
